## Europäisches Patentamt
## European Patent Office
## Office européen des brevets

(19)

(11) Veröffentlichungsnummer: **0 001 630**
**B1**

(12)
# EUROPÄISCHE PATENTSCHRIFT

(45) Veröffentlichungstag der Patentschrift: **16.09.81**

(21) Anmeldenummer: **78101165.5**

(22) Anmeldetag: **17.10.78**

(51) Int. Cl.³: **F 16 C 11/04, A 61 F 1/00, E 05 D 1/04**

(54) Scharniergelenk.

(30) Priorität: **26.10.77 DE 2747926**

(43) Veröffentlichungstag der Anmeldung:
**02.05.79 Patentblatt 79/9**

(45) Bekanntmachung des Hinweises auf die Patenterteilung:
**16.09.81 Patentblatt 81/37**

(84) Benannte Vertragsstaaten:
**DE FR GB**

(56) Entgegenhaltungen:
**CH - A - 542 624**
**DE - A - 1 708 218**
**DE - A - 2 614 170**
**DE - U - 7 517 440**
**GB - A - 1 007 660**
**US - A - 3 256 640**

(73) Patentinhaber: **HOECHST AKTIENGESELLSCHAFT**
**Postfach 80 03 20**
**D-6230 Frankfurt/Main 80 (DE)**

(72) Erfinder: **Zimmermann, Josef**
**Auf der Krautweide 11**
**D-6231 Sulzbach (DE)**

Courier Press, Leamington Spa, England.

## Scharniergelenk

Gegenstand der Erfindung ist ein Scharniergelenk aus gleichen Scharnierhälften, die mit Anschlagflächen versehen und durch mindestens einen, vorzugsweise zwei Ringe gelenkig verbunden und geführt sind und Nuten zur Aufnahme der Ringe aufweisen, die konzentrisch zur gedachten Drehachse angeordnet sind.

Scharniergelenke der eingangs genannten Art sind bekannt.

Nach der GB-A-1 007 660 werden Die Scharnierhälften durch Ringe, die Teile von Lagerabdeckungen bilden, gelenkig verbunden. Die Ringe und die Nuten zur Aufnahme der Ringe sind kegelstumpfförmig ausgebildet, damit beim Anziehen des Gelenkbolzens durch Einziehen der Ringe in die Nuten ein bestimmter Abstand der Gelenkköpfe zur zylindrischen Oberfläche der Nabe eingestellt werden kann. Bei dieser Scharnierverbindung ist ein Gelenkbolzen mit entsprechenden Spanneinrichtungen erforderlich, wodurch die Montage des Gelenks erheblich erschwert wird.

Nach der US-A-3 256 640 werden die Scharnierhälften durch ein segmentartiges Verbindungselement verbunden. Dabei muß das Verbindungselement, das in hinterschnittenen Nuten geführt wird, die sich in den Scharnierköpfen befinden, mit einer Scharnierhälfte fest verbunden werden, um ein Auswinanderfallen des Gelenkes während des Betriebs zu vermeiden.

Aufgabe zu vorliegender Erfindung ist ein Scharniergelenk der genannten Art zu schaffen, bei dem die Scharnierhälften weder durch Gelenkbolzen, festfixierte Führungssegmente noch durch Filmscharniere gelenkig verbunden sind. Ferner soll das Scharniergelenk eine gewisse Beweglichkeit quer und parallel zur Drehachse aufweisen.

Die Aufgabe wird durch ein Scharniergelenk gelöst, das dadurch gekennzeichnet ist, daß die Scharnierhälften jeweils einen, die Anschlagflächen bildenden, segmentförmigen Gelenkkopf aufweisen, die Ringe in Nuten mit Hinterschneidungen geführt sind und die Gelenkköpfe Zapfen als radiale Befestigungselemente aufweisen, die mit einem Schlitz und einer den Gelenkkopt durchdringenden Bohrung zur Aufnahme eines Spreizelements versehen sind.

Die Ausnehmungen an den Gelenkköpfen können entsprechend dem gewünschten Schwenkbereich des Scharniergelenks gewählt werden. 35 % eines jeden Ringes sollen jedoch mindestens in den Nuten der Gelenkköpfe geführt werden. Über eine Spielvorgabe der Führungsringe läßt sich eine gewisse Beweglichkeit der Gelenkteile quer und parallel zur Drehachse erreichen, was für den Ausgleich von Fluchtfehlern zwischen den zu verbindenden Gelenkteilen von Vorteil ist. Die Ringe können über Stege mit Abdeckungen verbunden sein. Für gewisse Anwendungsfälle kann es vorteilhaft sein, die Zapfen dübelartig und/oder poros zu gestalten, so daß sie sich besonders gut den Ausnehmungskonturen, in die sie eingesetzt werden sollen, durch spanende oder spanlose Formgebung anpassen lassen. Die poröse Struktur des Zapfens ermöglicht auch eine mickroformschlüssige Verbindung durch Kleben oder bei medizinischer Anwendung durch Anwachsen.

Die Verwendung des erfindungsgemäßen Scharniergelenks ist in allen Bereichen der Technik möglich. Es eignet sich Besonders vorteilhaft zum Verbinden von rohrförmigen Elementen. Hierbei kommt ihm die dübelartige Ausgestaltung seiner Befestigungselemente und das Fehlen jeglicher Achsstifte oder Bolzen zugute, da das Spreizen der dübelartigen Zapfen und damit das Justieren und Befestigen des Scharniergelenkes zu jeder Zeit, d.h. auch nach Verbinden der beiden Scharnierhälften vorgenommen werden kann. Wegen dieser Eigenschaft sowie wegen der porösen Struktur seiner Zapfen und der Möglichkeit seiner flachen Bauweise in Achsrichtung eignet sich das Scharniergelenk auch als Gelenk-Prothese für einachsige Gelenke bei Mensch und Tier.

Die Figuren zeigen das erfindungsgemäße Scharniergelenk in beispielsweiser Ausführung.

Fig. 1 zeigt die Seitenansicht des Scharniergelenks im gestreckten Zustand.

Figur 2 zeigt einen axialen Längsschnitt durch das Scharniergelenk.

Figur 3 zeigt die Einzelheit "Z" von Fig. 2 (Führungsring in hinterschnittener Nut).

Figur 4 zeigt das abgewinkelte, montierte Scharniergelenk als Verbindung zweier Rohre.

Figur 5 zeigt den Schnitt V—V durch den dübelförmigen Scharnierzapfen im montierten Zustand.

Figur 6 zeigt den Ausschnitt VI—VI von Figur 2.

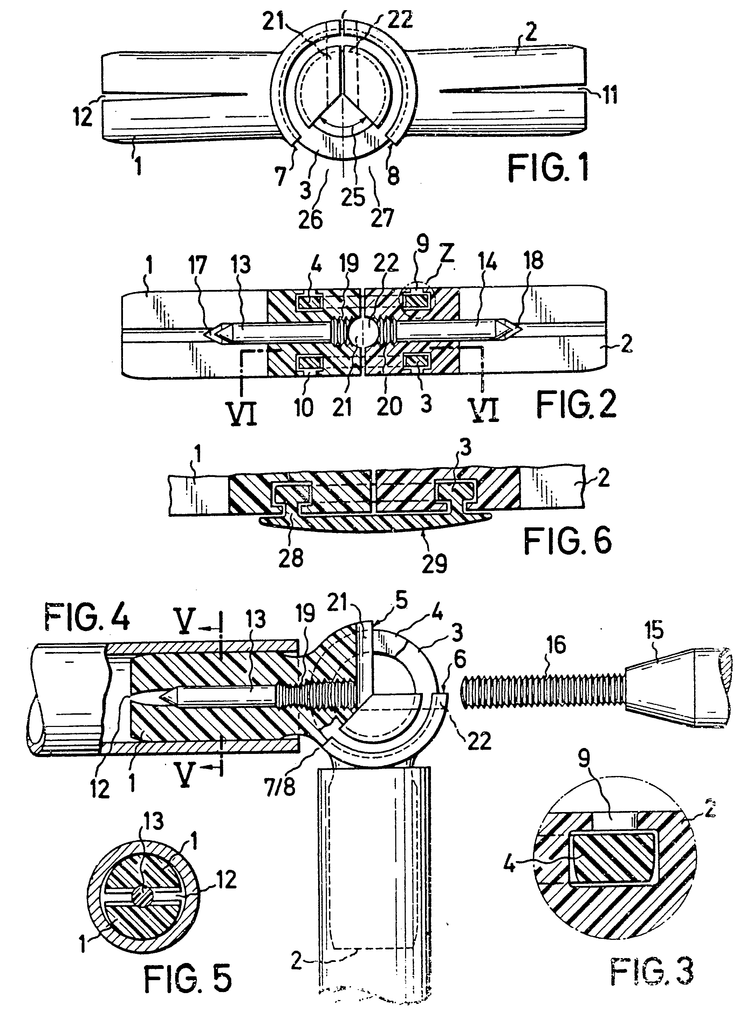

Bei dem in den Figuren 1 bis 5 dargestellten Ausführungsbeispiel sind die Scharnierhälften durch zwei Ringe 3 und 4 miteinanader verbunden. Der Schwenkwinkel des Scharniergelenks ist durch die Anschlagflächen 5 und 6 sowie 7 und 8 vorgegeben. Die Ringe 3 und 4 sind in hinterschnittene Nuten 9 und 10 eingeklipst. Das Scharniergelenk hat zwei dübelförmig spreizbare Zapfen 1 und 2 als Befestigungselemente mit den Schlitzen 11 und 12. Die Zapfen 1 und 2 werden durch die Spreizelemente 13, 14 aufgeweitet, indem sie mit einem Werkzeug 15 mit Gewindestift 16 in die Zapfenbohrungen 17, 18 eingedrückt werden. Der Gewindestift 16 läuft in den Gewinden 19, 20. Die Gewinde 19, 20 können vorgeschnitten sein oder durch das Einschrauben des Werkzeugs 15 geformt werden. Die Kanäle 21, 22 im Gelenkkopf ermöglichen das Justieren un Befestigen des Scharniergelenks im ungeteilten Zustand. Für vershiedene Anwendungen, insbesondere im medizinischen Bereich kann es zweckmäßig sein, die Stirnseiten Der Gelenkköpfe abzudecken. Zu diesem Zwecke können die Ring 3 und 4 über Stege 28 mit Abdeckungen 29 verbunden sein.

Beide Scharnierhälften werden als ein einziger Preßrohling hergestellt. Dabei kann das die Gelenkköpfe bildende Teil prismatische oder zylindrische Gestalt haben. Der Rohling wird anschließend unter Berücksichtigung des Schwenkwinkels 25 und der Lage der Anschläge 5, 6, 7 und 8 mit z.B. einer Messerstanze getrennt, wobei auch die Ausnehmungen 26 und 27 enstehen. Anschließend werden die Ringe 3 und 4 in die hinterschnittenen Nuten 9 und 10 geklipst und die Zapfenbohrungen 17 und 18 für die Spreizelemente 13 und 14 hergestellt. Dabei entstehen gleichzeitig die Kanäle 21 und 22. Die Gewinde 19 und 20 zum Eintreiben der Spreizelemente 13 und 14 können bei der Montage vom Werkzeug 15 gedrückt werden. Das Schlitzen der Zapfen 1 und 2 an den Scharnierhälften kann unmittelbar vor der Montage unter Berücksichtigung der Form der Ausnehmungen in den zu verbindenden Teilen mit einer Schneidvorrichtung vorgenommen werden.

Zur Herstellung des Scharniergelenks eignen sich Metalle, Kunststoffe und insbesondere sinterfähiges pluverförmiges Material, wie z.B. verpressbare sinterfähige Kunststoffe, Kunstharze, keramische Massen und Metalle.

Der Scharniergelenkrohling wird bildsam geformt, indem sinterfähiges Pulver in einer Form definiert verdichtet und anschließend erwärmt wird. Um einen kompakten Werkstoff im Bereich des Gelenkkopfes und einen porösen im Bereich der Befestigungselemente zu erhalten, wird das Pulver im Bereich des späteren Gelenkkopfes stärker verdichtet als im Bereich der Befestigungselemente. Bei Verwendung von Kunststoffpulver erfolgt die Wärmebehandlung oberhalb des Kristallitschmelzbereichs. Dabei schmilzt das hoch verdichtete Pulver zu kompaktem Werkstoff zusammen, während sich die Pulverkörner in weniger hoch verdichteten Bereichen zu einem porösen Werkstoff verbinden. Die Werkstoffeigenschaften an verschiedenen Stellen des Rohlings werden ausschließlich durch die unterschiedliche Verdichtung des Pulvers gesteuert; eine untershiedliche Temperaturführung an verschiedenen Stellen der Form ist nicht erforderlich.

## Patentansprüche

1. Scharniergelenk aus gleichen Scharnierhälften, die mit Anschlagflächen (7, 8) versehen und durch mindestens einen, vorzugsweise zwei Ringe (3, 4) gelenkig verbunden und geführt sind und Nuten (9, 10) zur Aufnahme der Ringe (3, 4) aufweisen, die konzentrisch zur gedachten Drehachse angeordnet sind, dadurch gekennzeichnet, daß die Scharnierhälften jeweils einen, die Anschlagflächen (7, 8) bildenden, segmentförmigen Gelenkkopf aufweisen, die Ringe (3, 4) in Nuten (9, 10) mit Hinterschneidungen geführt sind und die Gelenkköpfe Zapfen (1, 2) als radiale Befestigungselemente aufweisen, die mit einem Schlitz (11, 12) und einer der Gelenkkopf durchdringenden Bohrung (17, 18) zur Aufnahme eines Spreizelementes (13, 14) versehen sind.

2. Scharniergelenk nach Anspruch 1, dadurch gekennzeichnet, daß die Gelenkköpfe jeweils Ausnehmungen derart aufweisen, daß mindestens 35 % eines jeden Ringes (3, 4) in den Nuten (9, 10) geführt werden.

3. Scharniergelenk nach Anspruch 1 oder 2, dadurch gekennzeichnet, daß das Befestigungselement eine mikroporige Struktur aufweist.

4. Scharniergelenk nach Anspruch 1 bis 3, dadurch gekennzeichnet, daß die Ringe (3, 4) über Stege (28) mit Abdeckungen (29) verbunden sind.

## Revendications

1. Articulation à charnière se composant de deux moitiés de charnière identiques, qui sont pourvues de surfaces de butée (7, 8) qui sont reliées de façon articulée et guidées par au moins un, et de préférence deux anneaux (3, 4), et qui comportent, pour recevoir ces anneaux (3, 4), des rainures (9, 10) qui sont disposées concentriquement à l'axe de rotation théorique, caractérisée en ce que les moitiés de charnière comportent chacune une tête d'articulation en forme de segment formant les surfaces de butée (7, 8) en ce que les anneaux (3, 4) sont guidés dans les rainures (9, 10) comportant des parties dépouillées et en ce que les têtes d'articulation comportent, comme éléments radiaux de fixation, des broches (1, 2) qui sont pourvues d'une fente (11, 12) ainsi que d'un perçage (17, 18), traversant la tête d'articulation et servant à recevoir un élément d'expansion (13, 14).

2. Articulation à charnière selon la revendication 1, caractérisée en ce que les têtes d'articulation comportent chacune des évidements de manière qu'au moins 35 % de chaque anneau (3, 4) soit engagé dans les rainures (9, 10).

3. Articulation à charnière selon l'une des revendications 1 ou 2, caractérisé en ce que les éléments de fixation présentent une structure microporeuse.

4. Articulation à charnière selon l'une quelconque des revendications 1 à 3 caractérisée en ce que les anneaux (3, 4) sont reliés au moyen de voiles (28), comportant des couvercles (29).

## Claims

1. Hinged joint consisting of identical hinge halves, which are provided with stop faces (7 8), and are jointedly connected and guided by at least one, and preferably two, rings (3, 4) and comprise grooves (9, 10) for receiving the rings (3, 4) which are arranged concentrically with respect to the imaginary axis of rotation, characterised in that each hinge half has a segmental joint head which forms the stop faces (7, 8), the rings (3, 4) are guided in undercut grooves (9, 10) and the joint heads have pins (1, 2) as radial fastening elements which are provided with a slit (11, 12) and with a bore (17, 18) which extends through the joint head and serves to receive an expanding-element (13, 14).

2. Hinged joint according to claim 1, characterised in that each joint head is provided with recesses in such a way that at least 35 % of each ring (3, 4) is guided in the grooves (9, 10).

3. Hinged joint according to claim 1 or 2, characterised in that the fastening element has a microporous structure.

4. Hinged joint according to claims 1 to 3, characterised in that the rings (3, 4) are connected to covers (29) by webs (28).

0 001 630

FIG.1

FIG.2

FIG.6

FIG.4

FIG.5

FIG.3